# EUROPEAN PATENT APPLICATION

(11) **EP 4 850 677 A1**
(43) Date of publication of application: **09.12.2026**
(21) Application number: 24960076.8
(22) Date of filing: 21.11.2024
(51) Int. Cl.: A61K 36/074, A61P 11/00

(54) **USE OF REISHI SPORE OIL FOR PREVENTING AND TREATING PULMONARY NODULES**

(30) Priority: 06.11.2024 CN 202411575483
(71) Applicant: Guangzhou Hanfang Pharmaceuticals Co., Ltd., Guangzhou, Guangdong 510970 (CN)
(72) Inventor: YUAN, Cheng, Guangzhou, Guangdong 510970 (CN); XU, Wendong, Guangzhou, Guangdong 510970 (CN); WANG, Guocai, Guangzhou, Guangdong 510970 (CN); LIU, Juyan, Guangzhou, Guangdong 510970 (CN); YANG, Zifeng, Guangzhou, Guangdong 510970 (CN); MAO, Yukang, Guangzhou, Guangdong 510970 (CN); WANG, Kexin, Guangzhou, Guangdong 510970 (CN); CAO, Lin, Guangzhou, Guangdong 510970 (CN); CAI, Hongfei, Guangzhou, Guangdong 510970 (CN); LIU, Min, Guangzhou, Guangdong 510970 (CN); HE, Yingying, Guangzhou, Guangdong 510970 (CN)
(74) Representative: Loyer & Abello
(86) International application number: PCT/CN2024/133398
(87) International publication number: WO 2026/097619

(57) **Abstract**

Disclosed is use of reishi spore oil in the preparation of a drug for preventing and treating nodular diseases. Animal experiments show that reishi spore oil has the effect of significantly inhibiting the formation and development of benign granulomatous nodules in the lungs of mice, and also possesses good safety. The reishi spore oil can be used for preparing a drug for preventing and treating the formation and development of pulmonary nodules. The reishi spore oil comprises one or more components of triglyceride, ergosterol, and vitamin E; and the reishi polysaccharide ingredient is not detected.

## Description

### TECHNICAL FIELD

The present invention relates to the field of medical technology, and specifically, to a use of *Ganoderma lucidum* (reishi) spore oil in preventing and treating pulmonary nodules.

### BACKGROUND

*Ganoderma lucidum*, as a treasure in the traditional Chinese medicine of China, has long been known as "magical herb". It is indicated for uneasiness, insomnia and palpitation, lung deficiency cough and asthma, consumptive disease with shortness of breath, and poor appetite. *Ganoderma lucidum* spores are ultrafine spores projected from pileus of *Ganoderma lucidum* during the mature growth stage. They are reproductive cells of *Ganoderma lucidum*, and possess all genetic active substances of *Ganoderma lucidum*. Modern pharmacological studies have shown that *Ganoderma lucidum* spores have effects such as enhancing immunity, lowering blood glucose, lowering blood lipids, anti-tumor, anti-hypoxia, and scavenging free radicals. *Ganoderma lucidum* spore oil is a lipid-soluble active substance obtained from *Ganoderma lucidum* spores through wall-breaking and supercritical carbon dioxide extraction. Studies have shown that *Ganoderma lucidum* spore oil has effects such as enhancing immunity and protecting the liver. Existing studies generally consider that the main active components of *Ganoderma lucidum* are *Ganoderma lucidum* polysaccharides and *Ganoderma lucidum* triterpenoids. Research on various effects of *Ganoderma lucidum* is also mainly focused on polysaccharides and triterpenoids. However, there are currently few reports on component studies and efficacy analysis of other components in the spore oil, such as triglyceride compounds that account for the largest proportion.

In recent years, with the widespread application of multi-slice computed tomography and extensive implementation of lung cancer screening, the detection rate of pulmonary nodules has significantly increased worldwide. An article published in the authoritative journal *JAMA*, through observation of patients with pulmonary nodules, found that at least 95% of pulmonary nodules are benign and have no cancerous potential. Most patients with pulmonary nodules can recover spontaneously and have a favorable prognosis; however, one-third of the patients will still progress to chronic diseases, eventually leading to pulmonary fibrosis, pulmonary hypertension, respiratory failure, and even death. Against the backdrop of the currently high incidence of respiratory tract infections caused by viruses and mycoplasma, the prevention and treatment of pulmonary nodules have become a focus of attention for the media and the public in recent years. Clinically, for benign pulmonary nodules that progress slowly or remain unchanged over a long period, follow-up observation is mainly adopted, and guidelines still lack recommendations for effective preventive and therapeutic drugs. Currently, clinical approaches for treating sarcoidosis are limited. Glucocorticoids are the first-line treatment, but long-term and high-dose use causes serious adverse reactions, and recurrence is likely to occur after drug withdrawal. Therefore, there remains a need to continue searching for safe and effective therapeutic drugs for treating pulmonary nodules.

### SUMMARY

An objective of the present invention is to provide a use of *Ganoderma lucidum* spore oil in preparation of a medicament for preventing and treating formation and progression of pulmonary nodules. The prevention and treatment of pulmonary nodules have become a focus of attention in the media and among the public in recent years; however, guidelines still lack recommendations for effective preventive and therapeutic drugs. Based on the above reasons and requirements, it has been discovered that *Ganoderma lucidum* spore oil has a novel property of preventing and treating the formation and progression of pulmonary nodules, thereby providing a novel use of *Ganoderma lucidum* spore oil as a medicament, namely, use in the preparation of a medicament for preventing or treating pulmonary nodules.

To solve the above technical problems, the present invention adopts the following technical solutions:
Provided is a use of *Ganoderma lucidum* spore oil in preparing a formulation for preventing and treating sarcoidosis.

It is found through animal model experiments that *Ganoderma lucidum* spore oil has a significant effect of inhibiting the formation and progression of benign granulomatous nodules in mouse lungs, while possessing good safety. Therefore, it can be used for preparing a medicament for preventing and treating the formation and progression of pulmonary nodules.

Furthermore, the current experimental modeling methods are not completely targeted to the lungs, and may also exert similar preventive and therapeutic effects on sarcoidosis in organs other than the lungs, which can be further developed and exploited.

Preferably, the sarcoidosis includes pulmonary nodules.

Preferably, the sarcoidosis includes benign granulomatous nodules.

Preferably, inducing factors of the sarcoidosis include one or more of microorganisms and related derivatives thereof, and exogenous particles.

Preferably, the microorganisms include one or more selected from the group consisting of bacteria, viruses, and mycoplasma; and the exogenous particles include one or more selected from the group consisting of carbon nanoparticles, metal particles, and smoke.

Preferably, the *Ganoderma lucidum* spore oil includes one or more components selected from the group consisting of triglycerides, ergosterol, and vitamin E.

Preferably, in the *Ganoderma lucidum* spore oil, a content of triglyceride components is greater than 60%, a content of ergosterol ranges from 0.1% to 0.5%, a content of vitamin E ranges from 0.5% to 4%, and no *Ganoderma lucidum* polysaccharide compounds are detected.

Preferably, in the *Ganoderma lucidum* spore oil, the content of the triglyceride components is greater than 90%.

Preferably, in the *Ganoderma lucidum* spore oil, a mass content of the vitamin E is 2%.

More preferably, in the *Ganoderma lucidum* spore oil, no *Ganoderma lucidum* triterpenoids are detected.

Provided is a formulation obtained by the above use.

Preferably, the formulation includes a medicament; and the medicament further includes a pharmaceutically acceptable carrier.

Preferably, the medicament includes one selected from the group consisting of a tablet, a capsule, a buccal tablet, a granule, a soluble granule, a pellet, a powder, an ointment, a Dan, a suspension, a solution, an emulsion, an injection, a suppository, a cream, a spray, a drop, and a patch.

Compared with the prior art, the implementation of the present invention offers the following beneficial effects:
It is reported that an immunological characteristic of sarcoidosis is excessive immunity mediated by CD4+ T cells. Therefore, corticosteroids are still used clinically as first-line therapeutic drugs. *Ganoderma lucidum* spore oil is generally considered to have an immune-enhancing effect. Multiple studies have reported that *Ganoderma lucidum* spore oil can upregulate the proportion of CD4+ T cells in peripheral blood lymphocytes, thereby enhancing the body's immune capability. However, the present invention unexpectedly found that *Ganoderma lucidum* spore oil with a triglyceride content greater than 60%, an ergosterol content ranging from 0.1% to 0.5%, and a vitamin E content ranging from 0.5% to 4% has a significant preventive and therapeutic effect on pulmonary nodules, and can markedly inhibit the formation and progression of pulmonary granulomas in mice with benign granulomatous pulmonary nodules. It has no significant impact on body weight or overall health status of mice, and no obvious adverse reactions are observed, indicating good safety. The *Ganoderma lucidum* spore oil of the present invention has beneficial effects of being usable for preparing a medicament for preventing and treating pulmonary nodules.

### BRIEF DESCRIPTION OF THE DRAWINGS

FIG. 1 shows an HPLC component detection result of *Ganoderma lucidum* spore oil according to an embodiment of the present invention;
FIG. 2 shows a TLC polysaccharide detection result of *Ganoderma lucidum* spore oil according to an embodiment of the present invention;
FIG. 3 shows Micro-CT tomographic scanning detection results of mouse lungs according to an embodiment of the present invention; and
FIG. 4 shows pathological detection results of mouse lung tissues according to an embodiment of the present invention.

### DETAILED DESCRIPTION OF THE EMBODIMENTS

To better illustrate the objectives, technical solutions, and advantages of the present invention, the present invention will be further described below with reference to the drawings and specific embodiments, so that those skilled in the art could better understand and implement the present invention. However, the embodiments provided are not intended to limit the present invention. Modifications or replacements made to the methods, steps, or conditions of the present invention without departing from the spirit and essence of the present invention shall fall within the scope of the present invention. Unless otherwise specified, the technical means used in the embodiments are conventional means well known to those skilled in the art.

### Example 1

### Preparation and Component Detection of Ganoderma lucidum Spore Oil

A method for preparing the *Ganoderma lucidum* spore oil used in the present invention is as follows:
*Ganoderma lucidum* spore powder was taken and subjected to wall-breaking, and then extracted with supercritical CO₂ to obtain a crude *Ganoderma lucidum* spore oil. An appropriate amount of silica gel was taken and dissolved in a mixed solution of petroleum ether and ethyl acetate in a volume ratio of 8.5-9.5:0.5-1.5, and the resultant was wet-packed into a column to obtain an elution column. The ratio of column diameter to column height of the elution column was 1:10-20, and the mass ratio of the crude *Ganoderma lucidum* spore oil to the silica gel was 1:10-40. The crude *Ganoderma lucidum* spore oil was taken and loaded onto the elution column, and eluted with the above mixed solution of petroleum ether and ethyl acetate under atmospheric pressure. Eluates were collected, combined, concentrated under reduced pressure to remove the solvents, and vacuum-dried. Vitamin E was added to make its mass content 2%, thereby obtaining the *Ganoderma lucidum* spore oil. Through separation and purification, a large amount of impurities and moisture were removed, and the obtained *Ganoderma lucidum* spore oil was clear and transparent, with stable component contents.

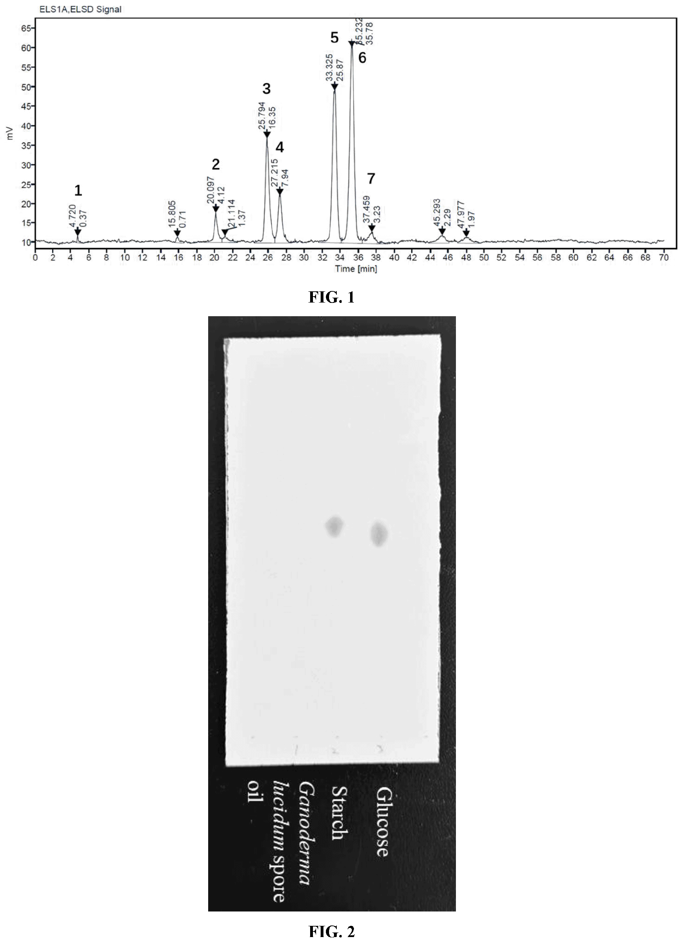

The contents of ergosterol and triglycerides in the *Ganoderma lucidum* spore oil were detected by high-performance liquid chromatography (HPLC). As shown in FIG. 1, peak 1 represents ergosterol, with a content of 0.37%; peaks 2-7 respectively represent different triglyceride components, with a total content of 93.29%; and no peak representing triterpenoid components was found.

Whether the *Ganoderma lucidum* spore oil contained polysaccharide components was detected by thin-layer chromatography (TLC), with starch and glucose selected as positive controls:
1. 10 g of the *Ganoderma lucidum* spore oil and 5 mL of water were added into a separatory funnel, thoroughly shaken, and allowed to stand for phase separation. An aqueous layer was separated, and an oil layer was discarded. The aqueous layer was concentrated under reduced pressure to 2 mL, and 6 mL of a strong acid was added. The resulting mixture was rapidly shaken, and placed in a water bath at 85 °C for 30 min. The resulting mixture was removed, and cooled to room temperature for subsequent use.
2. 10 g of starch was taken to prepare a 10% starch solution. 2 mL of the starch solution was mixed with 6 mL of a strong acid, rapidly shaken, and placed in a water bath at 85 °C for 30 min. The resulting mixture was removed, and cooled to room temperature for subsequent use.
3. 10 mg of anhydrous glucose was taken to prepare a glucose solution containing 1 mg of glucose per 1 mL. 2 mL of the glucose solution was mixed with 6 mL of a strong acid, rapidly shaken, and placed in a water bath at 85 °C for 30 min. The resulting mixture was removed, and cooled to room temperature for subsequent use.

5 µL of each of the above three solutions after reaction with the strong acid was separately taken and spotted onto the same silica gel thin-layer plate, developed with methanol as a developing solvent, and dried at 105 °C, and the spots thereof were observed, as shown in FIG. 2. After hydrolysis, both the starch solution and the glucose solution in the positive control groups exhibited spots representing monosaccharides, while no spot was showed at the corresponding position of the *Ganoderma lucidum* spore oil, indicating that no saccharide components (including polysaccharides, disaccharides, and monosaccharides) were detected in the *Ganoderma lucidum* spore oil.

### Example 2

Efficacy Study of *Ganoderma lucidum* Spore Oil on Mice with Benign Granulomatous Pulmonary Nodules

### 1. Experimental animals

Specific pathogen-free (SPF)-grade C57BL/6J mice (male, 6-8 weeks old), housed in an SPF-grade animal facility, with 12 h light/12 h darkness, at a temperature of 22 ± 2 °C, and a humidity of 50 ± 5%.

### 2. Experimental materials

Incomplete Freund's Adjuvant (IFA), *Mycobacterium tuberculosis* superoxide dismutase A (SodA), agarose beads, and the like were used for model establishment.

*Ganoderma lucidum* spore oil: *Ganoderma lucidum* spore oil prepared according to Example 1.

### 3. Experimental methods

### (1) Establishment of mouse models of benign granulomatous pulmonary nodules and administration

The mice were randomly divided into four groups: a blank group, a model group, a low-dose *Ganoderma lucidum* spore oil group, and a high-dose *Ganoderma lucidum* spore oil group, with 12 mice in each group. Administration began 30 days before modelling. The blank group and the model group were administered vehicle soybean oil, and the mice in the *Ganoderma lucidum* spore oil groups were intragastrically administered *Ganoderma lucidum* spore oil. Administration doses in the low-dose and high-dose groups were 0.3 g/kg and 1.2 g/kg, respectively. The administration volume in each group was 5 mL/kg. Administration was performed once daily for 51 consecutive days, and body weight changes were recorded.

On day 31, the model group and the *Ganoderma lucidum* spore oil groups were subcutaneously injected with an emulsion of SodA in IFA, the blank group was subcutaneously injected with an emulsion of IFA, and administration was continued. On day 44, the model group and the *Ganoderma lucidum* spore oil groups were injected via the tail vein with agarose beads conjugated with SodA, the blank group was injected with phosphate-buffered saline (PBS), and administration was continued until day 51.

### (2) Micro-CT imaging examination of mouse lungs

Before sampling, the mice in each group were anesthetized with isoflurane gas. Respiration of the mice was observed. After respiration became stable for several minutes, the mice were placed into a small-animal Micro-CT instrument and fitted with a respiratory mask. Instrument parameters were adjusted. The scanning position was set, and the camera was calibrated. The respiratory status and body posture of the mice were observed. After confirming the thoracic position and respiratory status of the mice, the tomographic scanning was performed to detect imaging changes in the mouse lungs.

Micro-CT statistics: Lungs of each mouse were maintained at the same window level. Regions having the same size were selected at each window level for each mouse to calculate CT values, followed by analysis.

### (3) Histopathological examination of mouse lung tissues

Fixation and embedding: Lung tissues were fixed with 4% paraformaldehyde, washed, and trimmed. The tissues were trimmed to obtain a flat section, dehydrated sequentially with graded ethanol solutions of 50%, 70%, 80%, 95%, and 100%, cleared by immersion sequentially in xylene I and II, infiltrated sequentially with paraffin I and II at 60 °C, and finally embedded in paraffin.

Sectioning and staining: The embedded tissues were cut into paraffin sections having a thickness of about 4 µm. The paraffin sections were deparaffinized by immersion sequentially in clearing agent I, clearing agent II, and clearing agent III, immersed in graded ethanol solutions of 100%, 90%, 80%, and 70% for 5 min each, washed with water and then stained with hematoxylin for 3 min, differentiated with 0.5% hydrochloric acid ethanol for several seconds, washed with tap water for 15 min for bluing and then stained with eosin for 2 min, dehydrated and cleared by immersion sequentially in 95% ethanol I, 95% ethanol II, 100% ethanol I, 100% ethanol II, clearing agent I, clearing agent II, and clearing agent III, and finally mounted with neutral gum.

Granuloma area statistics: The sections were observed and photographed under a microscope, and granuloma areas in the lung pathological images were statistically analyzed using ImageJ software.

### 4. Experimental results

During the experimental period, animals in each group exhibited good general conditions. The detection results of animal body weight are shown in Table 1. After modelling by SodA injection, the body weight of the mice decreased to a certain extent one day after the modelling and then gradually recovered. Compared with the blank group, the body weight of the model group was reduced, but there was no significant difference. *Ganoderma lucidum* spore oil showed no obvious effect on improving body weight loss in model mice with benign granulomatous pulmonary nodules. At the time of sampling, there was no significant difference in body weight of the animals among the groups.

**Table 1 Animal Body Weight Detection Results (g)**

| Group | Before administration | After first modelling | Before sampling |
|---|---|---|---|
| Blank group | 22.88 ± 0.60 | 26.21 ± 1.20 | 26.28 ± 0.94 |
| Model group | 23.09 ± 0.52 | 26.00 ± 1.05 | 26.45 ± 1.16 |
| Low-dose *Ganoderma lucidum* spore oil group | 22.77 ± 0.59 | 25.33 ± 0.88 | 25.86 ± 1.36 |
| High-dose *Ganoderma lucidum* spore oil group | 22.85 ± 0.62 | 24.80 ± 1.46 | 25.32 ± 1.02 |

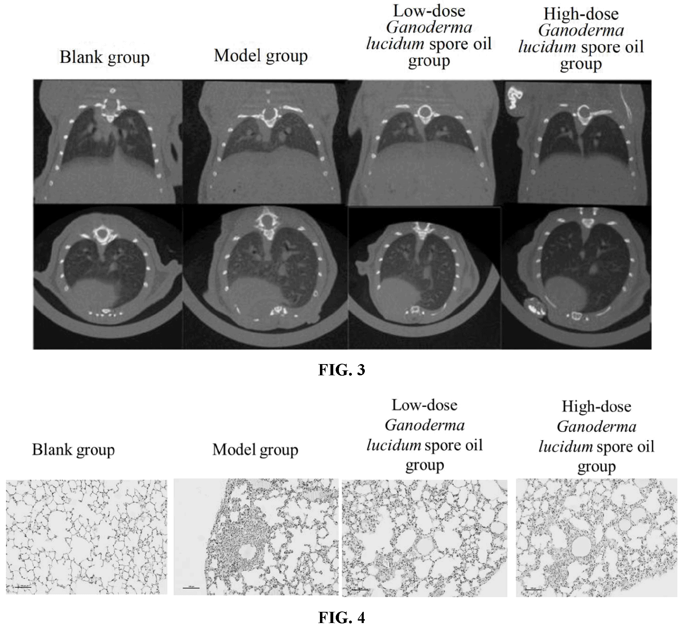

The results of the effect of *Ganoderma lucidum* spore oil on pulmonary imaging of mice with granulomatous pulmonary nodules are shown in FIG. 3. Observation of the pulmonary imaging results revealed that, compared with the blank group, the model group exhibited gray ground-glass-like opacities at different positions of the mouse lungs; the images of the low-dose and high-dose *Ganoderma lucidum* spore oil groups showed reduced gray ground-glass-like opacities. The CT value is a corresponding value in a CT image that is equivalent to an X-ray attenuation coefficient of each tissue. By scanning the mouse lungs with X-rays, different tissue components in the lungs have different X-ray absorption capabilities, resulting in different CT values. Compared with normal mice, mice with benign granulomatous pulmonary nodules have higher CT values. The CT values of each group after statistical analysis are shown in Table 2. Compared with the blank group, the CT value of the model group was significantly increased (*P* < 0.01). Compared with the model group, the CT values of the low-dose and high-dose *Ganoderma lucidum* spore oil groups were significantly decreased (*P* < 0.05 or *P* < 0.01), with statistically significant differences, indicating that the low dose and high dose of *Ganoderma lucidum* spore oil have an improving effect on granulomatous pulmonary nodules.

**Table 2 Statistical Results of Micro-CT Tomographic Scanning of Mouse Lungs**

| Group | CT value |
|---|---|
| Blank group | -763.35 ± 28.27 |
| Model group | -717.23 ± 22.65** |
| Low-dose *Ganoderma lucidum* spore oil group | -756.53 ± 24.49^{#} |
| High-dose *Ganoderma lucidum* spore oil group | -764.08 ± 43.52^{##} |

| | |
|---|---|
| Note: ***P* < 0.01 Compared with the blank group; ^{#}*P* < 0.05 and ^{##}*P* < 0.01 compared with the model group. | |

The results of the effect of *Ganoderma lucidum* spore oil on pulmonary pathology of mice with granulomatous pulmonary nodules are shown in FIG. 4. In the blank group, the alveolar tissues were intact without abnormalities. In the model group, a plurality of granulomas were visible around agarose beads in the lung tissues, centers of the granulomas were composed of epithelioid-like cells, and a large number of lymphocytes surrounded the periphery of the granulomas. Compared with the model group, the *Ganoderma lucidum* spore oil groups showed a certain ameliorating effect on the granuloma areas in the mouse lungs, with only a thin layer of epithelioid-like cells surrounding naked beads, and less lymphocyte infiltration around. The statistical results of granuloma areas are shown in Table 3. Compared with the model group, the lung granuloma area in the low-dose *Ganoderma lucidum* spore oil group was significantly reduced (*P* < 0.05), with a statistically significant difference. The granuloma area *in the* high-dose *Ganoderma lucidum* spore oil group also showed a trend of reduction to a certain extent, indicating that the low dose and high dose of *Ganoderma lucidum* spore oil have an ameliorating effect on granulomatous pulmonary nodules.

**Table 3 Statistical Results of Granuloma Areas in Mouse Lung Tissues**

| Group | Granuloma area (mm²) |
|---|---|
| Model group | 0.013 ± 0.001 |
| Low-dose *Ganoderma lucidum* spore oil group | 0.010 ± 0.001* |
| High-dose *Ganoderma lucidum* spore oil group | 0.011 ± 0.002 |

| | |
|---|---|
| Note: Compared with the model group, **P* < 0.05. | |

The above study, by establishing the mouse models of granulomatous pulmonary nodules and using techniques such as Micro-CT imaging and pathological analysis, demonstrates that *Ganoderma lucidum* spore oil with a triglyceride content greater than 60%, an ergosterol content ranging from 0.1% to 0.5%, and a vitamin E content ranging from 0.5% to 4% can alleviate progression of pulmonary nodules by reducing the formation and growth of granulomas in mice with granulomatous pulmonary nodules.

In summary, through the experiments and studies on *Ganoderma lucidum* spore oil with a triglyceride content greater than 60%, an ergosterol content ranging from 0.1% to 0.5%, and a vitamin E content ranging from 0.5% to 4%, the present invention has found that *Ganoderma lucidum* spore oil has the effect of preventing and treating the formation and progression of pulmonary nodules, and can significantly reduce the growth of granulomas in mice with granulomatous pulmonary nodules. It has no significant effects on body weight or overall health status of the mice, indicating that *Ganoderma lucidum* spore oil has good safety. The *Ganoderma lucidum* spore oil of the present invention has the beneficial effect of being usable for preparing a medicament for preventing or treating pulmonary nodules.

The above merely discloses preferred embodiments of the present invention and should not be construed as limiting the scope of the present invention. Therefore, equivalent changes made according to the claims of the present invention shall still fall within the scope of the present invention.

## Claims

1. A use of *Ganoderma lucidum* spore oil in preparing a formulation for preventing and treating sarcoidosis.

2. The use according to claim 1, **characterized in that** the sarcoidosis comprises pulmonary nodules.

3. The use according to claim 1, **characterized in that** the sarcoidosis comprises benign granulomatous nodules.

4. The use according to claim 1, **characterized in that** inducing factors of the sarcoidosis comprise one or more of microorganisms and related derivatives thereof, and exogenous particles.

5. The use according to claim 4, **characterized in that** the microorganisms comprise one or more selected from the group consisting of bacteria, viruses, and mycoplasma; and the exogenous particles comprise one or more selected from the group consisting of carbon nanoparticles, metal particles, and smoke.

6. The use according to claim 1, **characterized in that** the *Ganoderma lucidum* spore oil comprises one or more components selected from the group consisting of triglycerides, ergosterol, and vitamin E.

7. The use according to claim 1, **characterized in that** in the *Ganoderma lucidum* spore oil, a content of triglyceride components is greater than 60%, a content of ergosterol ranges from 0.1% to 0.5%, a content of vitamin E ranges from 0.5% to 4%, and no *Ganoderma lucidum* polysaccharide compounds are detected.

8. A formulation obtained by the use according to claim 1.

9. The formulation according to claim 8, **characterized in that** the formulation comprises a medicament; and the medicament further comprises a pharmaceutically acceptable carrier.

10. The formulation according to claim 9, **characterized in that** the medicament comprises one selected from the group consisting of a tablet, a capsule, a buccal tablet, a granule, a soluble granule, a pellet, a powder, an ointment, a Dan, a suspension, a solution, an emulsion, an injection, a suppository, a cream, a spray, a drop, and a patch.
